# EUROPEAN PATENT APPLICATION

(11) **EP 4 299 730 A1**
(43) Date of publication of application: **03.01.2024**
(21) Application number: 22759816.6
(22) Date of filing: 25.02.2022
(51) Int. Cl.: C12N 9/04, A23C 9/127, A23L 5/00

(54) **METHOD FOR PRODUCING FERMENTED FOOD OR BEVERAGE, AND ANAEROBIC FERMENTATION METHOD**

(30) Priority: 26.02.2021 JP 2021031032; 18.08.2021 JP 2021133303
(71) Applicant: Amano Enzyme Inc., Nagoya-shi Aichi 460-8630 (JP)
(72) Inventor: KAMON Masahiro, Kakamigahara-shi, Gifu 509-0109 (JP); TADA Shusaku, Kakamigahara-shi, Gifu 509-0109 (JP); HASHIMURA Dai, Kakamigahara-shi, Gifu 509-0109 (JP); YAMASHIRO Kan, Kakamigahara-shi, Gifu 509-0109 (JP); HAYASHI Takuma, Kakamigahara-shi, Gifu 509-0109 (JP)
(74) Representative: D Young & Co LLP
(86) International application number: PCT/JP2022/007979
(87) International publication number: WO 2022/181782

(57) **Abstract**

The main purpose of this technique is to newly provide a technique whereby it becomes possible to shorten the time for anaerobic fermentation by reducing the concentration of oxygen contained in a raw material by a method other than an inert gas replacement method that requires the introduction of a facility. This technique provides a method for producing a fermented food or beverage, including a saccharide oxidase action step for allowing a saccharide oxidase to act on a portion or the whole of a saccharide in a raw material, and an anaerobic fermentation step for performing anaerobic fermentation. This technique also provides an oxygen concentration reducing agent for anaerobic fermentation use which contains a saccharide oxidase. This technique further provides an anaerobic fermentation method which includes a saccharide oxidase action step for allowing a saccharide oxidase to act on a portion or the whole of a saccharide in a raw material.

## Description

### Technical Field

The present invention relates to a method for promoting an anaerobic fermentation. More specifically, the present invention relates to a production technique for shortening the time for anaerobic fermentation by reducing the concentration of oxygen contained in a raw material using a saccharide oxidase.

### Background Art

It is disclosed in Non-Patent Literature 1, Patent Literatures 1 to 3 and the like that the fermentation efficiency is improved and the time for fermentation of fermented milk can be shortened by replacing and reducing dissolved oxygen in a raw material milk with an inert gas such as nitrogen. It is also possible to remove the oxygen dissolved in the raw material milk by deaeration instead of inert gas introduction. Also in Patent Literature 4, a method for producing a fermented milk which employs a yeast lactase in combination with a step for reducing the concentration of oxygen contained in a raw material milk is disclosed and purported to be capable of maintaining the flavor and the quality constantly regardless of the conditions of the lactic acid bacterium and the enzyme.

### Citation List

### Patent Literature

Patent Literature 1: Japanese non-examined patent publication No.2005-176603
Patent Literature 2 : Japanese non-examined patent publication No.2005-348703
Patent Literature 3 : Japanese non-examined patent publication No.2007-104995
Patent Literature 4 : WO2012/026384, Pamphlet

### Non-Patent Literature

Non-Patent Literature 1: Seibutsu-kogaku Kaishi Vol. 90, pages 335-339

### Summary of Invention

### Technical Problem

While the techniques for improving the fermentation efficiency are going to be developed diversely as described above, the oxygen concentration reducing techniques of prior art are problematic because they require large-scaled facilities enabling inert gas replacement such as a nitrogen replacement instrument.

Accordingly, the main purpose of this technique is to newly provide a technique whereby it becomes possible to shorten the time for anaerobic fermentation by reducing the concentration of oxygen contained in a raw material by a method other than an inert gas replacement method that requires the introduction of a facility. Solution to Problem

Applicants discovered, through diligent research in a technique capable of shortening the time for anaerobic fermentation, that by allowing a saccharide oxidase to act on a raw material milk it becomes possible to shorten the time for anaerobic fermentation, and finally established the present invention.

Thus, this technique firstly provides a method for producing a fermented food or beverage, including a saccharide oxidase action step for allowing a saccharide oxidase to act on a portion or the whole of a saccharide in a raw material; and, an anaerobic fermentation step for performing anaerobic fermentation.

In the method for producing a fermented food or beverage according to this technique, the aforementioned saccharide oxidase action step can be performed before the aforementioned anaerobic fermentation step and/or simultaneously with the aforementioned anaerobic fermentation step.

In this technique, a saccharide oxidase having a property of acting on one or more saccharides selected from glucose, maltotriose, maltose, galactose, maltotetraose, lactose, cellobiose and maltodextrin can be used as the aforementioned saccharide oxidase.

Also in this technique, as the aforementioned saccharide oxidase, it is possible to use a saccharide oxidase consisting of a polypeptide represented by any of the following (1) to (3):
(1) a polypeptide consisting of the amino acid sequence represented by SEQ ID NO:1,
(2) a polypeptide which is formed as a result of substitution, addition, insertion or deletion of one or several amino acid residues in the amino acid sequence represented by SEQ ID NO:1 and which exhibits a substrate specificity equivalent to that of the polypeptide consisting of the amino acid sequence represented by SEQ ID NO:1; and,
(3) a polypeptide which has a sequence identity of 90% or more to the amino acid sequence represented by SEQ ID NO: 1 in the amino acid sequence represented by SEQ ID NO:1 and which exhibits a substrate specificity equivalent to that of the polypeptide consisting of the amino acid sequence represented by SEQ ID NO:1.

In a method for producing a fermented food or beverage according to this technique, a lactic acid fermentation can be performed in the aforementioned anaerobic fermentation step.

In a method for producing a fermented food or beverage according to this technique, a fermented milk can be produced as the aforementioned fermented food or beverage.

This technique secondly provides an oxygen concentration reducing agent for anaerobic fermentation use which contains a saccharide oxidase.

This technique further provides an anaerobic fermentation method which includes a saccharide oxidase action step for allowing a saccharide oxidase to act on a portion or the whole of a saccharide in a raw material.

In the anaerobic fermentation method according to this technique, the aforementioned saccharide oxidase action step can be performed before an anaerobic fermentation step and/or simultaneously with the anaerobic fermentation step. Advantageous Effects of Invention

According to this technique, the time for anaerobic fermentation can be shortened with no need of introducing a facility by means of performing a treatment for reducing the concentration of oxygen contained in a raw material using a saccharide oxidase in the anaerobic fermentation.

### Description of Embodiments

Preferred embodiments for implementing the present invention are described below. The following embodiments are merely representatives of the embodiment of the invention, by which the scope of the present invention is not interpreted narrowly.

### <1. Method for producing fermented food or beverage>

The method for producing a fermented food or beverage according to this technique is a method in which a saccharide oxidase action step and an anaerobic fermentation step are at least performed. Otherwise, it is also possible to perform an ordinary food producing step before or after each step or simultaneously with each step depending on the type of the fermented food or beverage or the like, as long as the advantageous effects of this technique are not affected adversely. Each step is detailed below.

### (1) Saccharide oxidase action step

The saccharide oxidase action step is a step for allowing a saccharide oxidase to act on a portion or the whole of a saccharide in a raw material. In this technique, it becomes possible to reduce the concentration of oxygen contained in a raw material by allowing the saccharide oxidase to act on the saccharide in the raw material. As a result, it becomes possible to shorten the time for anaerobic fermentation in the anaerobic fermentation step described below.

Also by allowing the saccharide oxidase to act on the raw material, it becomes possible to improve the spinnability of the fermented food or beverage obtained via the anaerobic fermentation step described below. In particular, in the case of fermenting a plant raw material, there has been a problem that the viscosity and the spinnability are sometimes inferior to those of a fermented food or beverage using an animal raw material such as cow milk. However, by using this technique, the spinnability of a fermented food or beverage using a plant raw material can also be improved. The saccharide oxidase which can be employed in this technique is detailed below.

As used in this technique, the "spinnability" means a nature of a material to "form a thread", and can be evaluated based on the time period until the termination of the flow of a flowing sample as measured for example by using Zahn Cup (manufactured by RIGO). An improved spinnability of a food or beverage gives a unique texture in the mouth.

### A. Saccharide oxidase

While the saccharide oxidase which can be employed in this technique is not limited particularly as long as it is an enzyme which can oxidize a saccharide, it is preferably an enzyme which oxidizes oligosaccharides which are disaccharides or higher saccharides. Those which can exemplified typically are proteins having the physicochemical characteristics described below.

### (A) Action

The saccharide oxidase which can be employed in this technique oxidizes a saccharide described below in the presence of oxygen to generate a saccharic acid. More specifically, when allowing the saccharide oxidase which can be employed in this technique to act on the saccharide described below in the presence of oxygen, the saccharic acid and hydrogen peroxide are generated.

### (B) Substrate specificity

The saccharide oxidase which can be employed in this technique may be a protein which exhibits activity on one or more saccharides selected from glucose, maltotriose, maltose, galactose, maltotetraose, lactose, cellobiose and maltodextrin. The relative activity on each substrate based on the activity on glycose regarded as 100% is about 92% on maltotriose, about 86% on maltose, about 79% on galactose, about 60% on maltotetraose, about 58% on lactose, about 53% on cellobiose, about 24% on maltodextrin.

In the present invention, when the relative activity is 50% or higher based on the activity (100%) when glucose is used as a substrate, it is judged that "this is a substrate that the enzyme acts well".

As discussed above, when using a saccharide oxidase which exhibits an activity not only on monosaccharides such as glucose but also on a wider range of saccharides which are disaccharides or higher saccharides, it becomes possible to allow the saccharide oxidizing action to be functioned in a wider range of the field where existing glucose oxidases or oligosaccharide oxidases are not effective.

### (C) Km Value

In this technique, the method for calculating Km value (Michaelis constant) of a protein is not limited particularly, and any known method can be employed for calculation. The method for calculating Km value of a protein may for example be Lineweaver-Burk plot, Eadie-Hofstee plot, Hanes-Woolf plot and the like, and preferably Hanes-Woolf plot. While the Km value of the saccharide oxidase which can be employed in this technique is not limited particularly, [Km value of glucose]/[Km value of maltose] ≤ 1 is preferable, and 0.4 ≤ [Km value of glucose]/[Km value of maltose] ≤ 1 is more preferable.

### (D) Molecular mass

The saccharide oxidase which can be employed in this technique may be a saccharide oxidase having a molecular mass of about 63 kDa when measured by SDS-PAGE method.

### (E) Optimum pH

The saccharide oxidase which can be employed in this technique may be a saccharide oxidase exhibiting the highest saccharide oxidase activity at a pH around 5.0 to 9.0 in a reaction condition of 37°C for 5 minutes.

### (F) Stable pH range

The saccharide oxidase which can be employed in this technique may be a saccharide oxidase which is stable at a pH around 5.0 to 10.5 in a treatment condition of 37°C for 15 minutes.

### (G) Optimum temperature

The saccharide oxidase which can be employed in this technique may be a saccharide oxidase exhibiting the highest saccharide oxidase activity at a temperature around 20°C to 55°C in a reaction condition of pH 7.0 for 5 minutes.

### (H) Temperature stability

The saccharide oxidase which can be employed in this technique may be a saccharide oxidase capable of maintaining an activity of 80% or higher even when treated in a condition of a temperature not higher than 45°C in a treatment condition of pH 7.0 for 15 minutes.

### (I) Origin

The origin of the saccharide oxidase which can be employed in this technique described above is not limited particularly, and may for example be those originated from microorganisms belonging to Acremonium genus. In such a case, the microorganism belonging to Acremonium genus may for example be Acremonium chrysogenum.

As used herein "saccharide oxidase originated from Acremonium chrysogenum" means a saccharide oxidase produced by a microorganism classified as Acremonium chrysogenum (which may be a wild strain or a mutated strain) or a saccharide oxidase obtained by a gene engineering method utilizing a saccharide oxidase gene. Accordingly, a recombinant enzyme produced by a host microorganism into which a saccharide oxidase gene obtained from Acremonium chrysogenum (or a gene modified from such a gene) was transduced is also a "saccharide oxidase originated from Acremonium chrysogenum".

Examples of Acremonium chrysogenum from which the saccharide oxidase which can be employed in this technique is originated include Acremonium chrysogenum NBRC30055 (NITE, Japan), ATCC15006 (ATCC, United States), and DSM880 (DSMZ, Germany).

### (J) Amino acid sequence

While the amino acid sequence of the saccharide oxidase which can be employed in this technique is not limited, it may for example be specified by the following amino acid sequence.

Typically, the saccharide oxidase which can be employed in this technique can be specified by the amino acid sequence represented by SEQ ID NO:1.

Here, in the case where a portion of the amino acid sequence of a certain protein is modified, the protein after modification sometimes has a function equivalent to that of the protein before modification. Thus, in some case the modification of the amino acid does not influence substantially on the function of the protein, and the function of the protein is maintained before and after the modification. Accordingly, in another aspect of the present invention, there is provided a protein which consists of an amino acid sequence formed as a result of deletion, substitution and/or addition of one to several amino acids in the amino acid sequence represented by SEQ ID NO:1 and which has a saccharide oxidase activity. "Deletion, substitution and/or addition of one to several amino acids constituting an amino acid sequence" typically means a difference in a portion of the amino acid sequence.

Here the difference in the amino acid sequence is acceptable as long as the saccharide oxidase activity is preserved (the activity may somewhat be changed). As long as this requirement is satisfied, the position of the difference in the amino acid sequence is not limited particularly, and the difference may occur at a plural number of positions. As used herein, the plural means a number less than about 30% of the entire amino acid sequence, preferably a number less than about 20%, more preferably a number less than about 10%, further preferably a number less than about 5%, and most preferably a number less than about 1%.

Thus, it means that there is an identity of for example about 70% or higher, preferably about 80% or higher, more preferably about 90% or higher, further preferably about 95% or higher, and most preferably about 99% or higher, with the amino acid sequence of SEQ ID NO: 1.

Also in a preferable method, a conservative amino acid substitution is allowed to occur in the amino acid residues not essential to the saccharide oxidase activity to obtain a protein. As used herein, "conservative amino acid substitution" means a substitution of a certain amino acid residue with an amino acid residue having a side chain of the same property. The amino acid residues are classified into several families based on their side chains, such as basic side chains (for example, lysine, arginine, histidine), acidic side chains (for example, aspartic acid, glutamic acid), uncharged polar side chains (for example, glycine, asparagine, glutamine, serine, threonine, tyrosine, cysteine), non-polar side chains (for example, alanine, valine, leucine, isoleucine, proline, phenylalanine, methionine, tryptophan), β-branched side chains (for example, threonine, valine, isoleucine), and aromatic side chains (for example, tyrosine, phenylalanine, tryptophan, histidine). The conservative amino acid substitution is preferably a substitution between amino acid residues in the same family.

The identity (%) between two amino acid sequences or two nucleic acids (hereinafter referred to collectively as "two sequences") can be determined by the following procedure. First, two sequences are aligned so that an optimum comparison can be made. A gap may be introduced here into the first sequence thereby optimizing the alignment with the second sequence. When the molecule (amino acid residue or nucleotide) at a specific position in the first sequence is identical to the molecule at the corresponding position in the second sequence, the molecules at the positions can be regarded as identical. The identity between the two sequences is a function of the number of identical positions existing commonly between these two sequences (i.e., identity (%) = number of identical positions/total number of positions × 100), and preferably the number and the size of gaps required for alignment optimization are also taken into consideration.

Comparison and identity determination between two sequences can be conducted using a mathematic algorithm. Specific examples of the mathematic algorithms utilizable for sequence comparison may be, but is not limited to, the algorithm described in Karlin and Altschul (1990) Proc.Natl.Acad.Sci. USA 87:2264-68 and modified in Karlin and Altschul (1993) Proc.Natl.Acad.Sci. USA 90:5873-77. Such an algorithm is incorporated into NBLAST program and XBLAST program (version 2.0) described in Altschul et al (1990) J.Mol.Biol. 215:403-10. In order to obtain a nucleotide sequence equivalent to the nucleic acid molecule of the present invention, BLAST nucleotide search may for example be conducted using NBLAST program with score = 100 and word length = 12.

In order to obtain an amino acid sequence equivalent to the polypeptide molecule of the present invention, BLAST polypeptide search may for example be conducted using XBLAST program with score = 50 and word length = 3. In order to obtain a gap alignment for comparison, Gapped BLAST described in Altschul et al. (1997) Nucleic Acids Research 25 (17) :3389-3402 can be utilized. When utilizing BLAST and Gapped BLAST, default parameters of the corresponding programs (for example, XBLAST and NBLAST) can be used. For more details, see http://www.ncbi.nlm.nih.gov.

Another mathematic algorithm utilizable for sequence comparison may for example be an algorithm described in Myers and Miller (1988) Comput Appl Biosci. 4:11-17. Such algorithm is incorporated into ALIGN program utilizable for example via GENESTREAM network server (IGH Montpellier, France) or ISREC server. When using ALIGN program for amino acid sequence comparison, PAM 120 residue mass table may for example be utilized with gap length penalty = 12 and gap penalty = 4.

The saccharide oxidase which can be employed in this technique may be a portion of a larger protein (for example, fusion protein). The sequence to be added in the fusion protein may for example be a sequence serving for purification such as multi histidine residue and an adduct sequence ensuring the stability during recombination production.

A protein having the amino acid sequence described above can readily be prepared by a gene engineering method. For example, it can be prepared by transforming a suitable host cell (for example, Escherichia coli, yeast, filamentous fungus) with a DNA encoding this protein and collecting a protein expressed in a transformant. The collected protein is prepared appropriately depending on the purpose. In such method for obtaining this protein as a recombinant protein, various modifications become possible. For example, by inserting a DNA encoding this protein and other suitable DNA into an identical vector and then using the resulting vector for producing a recombinant protein, it becomes possible to obtain this protein consisting of the recombinant protein in which an optional peptide or protein is ligated. It is also possible to perform modification so that addition of a saccharide chain and/or lipid or N-terminal or C-terminal processing occurs. As a result of such modification, the recombinant protein can be extracted or purified more easily or a biological function can be added.

The saccharide oxidase action step may employ respective conditions which can be set as desired as long as the advantageous effects of this technique is not affected adversely. For example, the pH, temperature and time for the action can be set depending on the physicochemical characteristics of the saccharide oxidase employed, such as optimum pH, stable pH range, optimum temperature, temperature stability and the like. The pH can be set for example at pH 5.0 to 10.5, and preferably pH 5.0 to 7.5. The temperature can be set for example at 20°C to 55°C, preferably 30°C to 50°C, and more preferably 35°C to 45°C. The time for the action can be set for example at 1 to 12 hours, and preferably 4 to 10 hours.

The amount of the saccharide oxidase in the saccharide oxidase action step can be set as desired as long as the advantageous effects of this technique is not affected adversely. For example, in case of intending to improve the fermentation time-shortening effects, addition to the raw material is made preferably at 0.005 U/mL or more, more preferably at 0.008 U/mL or more, further preferably at 0.08 U/mL or more, and even further preferably at 0.17 U/mL or more. In case of intending to improve the spinnability of a fermented food or beverage, the saccharide oxidase is added preferably at 1.5 U/mL or more.

In the saccharide oxidase action step, other enzymes can also be used simultaneously in addition to the saccharide oxidase, as long as the advantageous effects of this technique is not affected adversely. For example, one or more enzymes selected from lactase, protease, transglutaminase, laccase, peroxidase, catalase, lipase, cellulase, and amylase can be employed simultaneously.

Also in the saccharide oxidase action step, a saccharide can be used simultaneously. Simultaneous use of the saccharide allows oxygen in a food or beverage raw material to be consumed when saccharide is oxidized, thereby improving the efficiency of deoxygenation. While the type of the saccharide which can be used simultaneously in this technique is not limited particularly as long as the advantageous effects of this technique is not affected adversely, one or more saccharide selected from starch, processed starch, dextrin, cellulose and processed cellulose can be used simultaneously.

### (2) Anaerobic fermentation step

The anaerobic fermentation step is step for fermenting a portion or the whole of a raw material under an anaerobic condition. The anaerobic fermentation step can be performed after the aforementioned saccharide oxidase action step, and can also be performed simultaneously with the aforementioned saccharide oxidase action step.

In the anaerobic fermentation step of this technique, an anaerobe can be employed to perform fermentation. The anaerobe which can be employed in this technique can be selected as desired as long as the advantageous effects of this technique is not affected adversely, and may be one or more anaerobes which can be employed for producing a general fermented food or beverage depending on the type of the fermented food or beverage to be produced. For example, the anaerobe may be lactobacillus, yeast and the like.

In the anaerobic fermentation step of this technique, lactic acid fermentation can be performed. The lactic acid bacterium employed in the lactic acid fermentation can be selected as desired as long as the advantageous effects of this technique is not affected adversely, and may be one or more lactic acid bacteria which can be employed for producing a general lactic acid-fermented food or beverage depending on the type of the lactic acid-fermented food or beverage to be produced. Examples of the lactic acid bacterium include lactic acid cocci belonging to Lactococcus, Streptococcus, Pediococcus, and Leuconostoc, lactic acid bacilli belonging to Lactobacillus, and Bifidobacterium, and preferred examples include Lactobacillus delbrueckii subsp. bulgaricus, Streptococcus thermophilus, and Lactobacillus delbrueckii subsp. delbrueckii.

While in this technique the aforementioned saccharide oxidase action step is performed to reduce the oxygen concentration contained in a raw material thereby promoting the progression of the anaerobic fermentation, performing an aerobic fermentation is not excluded. For example, before performing the aforementioned saccharide oxidase action step, an aerobic fermentation using an aerobe can be performed, and the aerobic fermentation step can be performed also simultaneously with or after the aforementioned saccharide oxidase action step or anaerobic fermentation step in case of a microorganism which is viable even in the presence of poor oxygen.

The respective conditions when performing the anaerobic fermentation step can be set as desired as long as the advantageous effects of this technique is not affected adversely. For example, fermentation condition, heating condition and the like can be set as desired depending on the type of the anaerobe employed. The fermentation temperature can be set for example at 20°C to 55°C, preferably 30°C to 50°C, and more preferably 35°C to 45°C. The time for fermentation can be set for example at 1 to 12 hours, and preferably 4 to 10 hours. The heating conditions may for example be high temperature short time pasteurization (HTST) method and ultrahigh temperature processing (UHT) method, retort method and the like, and the condition allowing the temperature of the food or beverage to reach 90°C or higher, and preferably about 95°C is acceptable. A method in which a food or beverage material is processed at 90 to 100°C for 1 to 5 minutes or at 90 to 95°C for 1 to 3 minutes may be exemplified.

By using a method for producing a fermented food or beverage described above, various fermented food or beverage can be efficiently produced. In this technique, a "fermented food or beverage" means a food or beverage obtained by fermenting a food or beverage material. Specifically, fermented milk (yogurt), lactic acid bacterium beverage, cheese, yogurt paste, liquors (Japanese sake, beer, wine, shochu and the like), various fermented seasonings (soy sauce, miso, vinegar and the like), rice bran pickles, kimchi, natto, arrowroot-starch mochi and the like may be exemplified. Also in this technique, a "fermented food or beverage" also includes a secondarily processed food or beverage made from such a fermented food or beverage.

In this technique, a "fermented milk" means an "animal fermented milk" originated from an animal and a "plant-base fermented milk" originated from a plant-base. The "animal fermented milk" means a "fermented milk" which is defined in "Ministerial ordinance concerning compositional standards, etc. for milk and milk products" as "the products which are obtained by fermenting milk, or milk, etc. containing an equal or greater amount of milk solids-not-fat with lactic acid bacteria or yeast and then forming a paste or liquid, or the frozen product". A "plant-base fermented milk" means a product formed by fermenting a plant-base milk, and the plant-base milk, may for example be a plant-base milk originated for example from green pea, soybean, fava bean, chick pea, barley, wheat, oat, rice, soba, barnyard millet, millet, hemp (industrial hemp), algae, almond, cashew nut, hazel nut, pecan nut, macadamia nut, pistachio, walnut, Brazil nut, peanut, and coconut.

In each step in a method for producing a fermented food or beverage according to this technique, a raw material employed for producing a general food or beverage can be selected and used as desired in addition to food or beverage materials (animal milks, plant-base milks, cereals, vegetables, fruits, peas, seafoods, meats and the like), various enzymes, various microorganisms, as long as the advantageous effects of this technique is not affected adversely. For example, various seasonings, pH modifiers, colorants, corrigents, stabilizers and the like can be employed.

### <2. Oxygen concentration reducing agent for anaerobic fermentation use>

An oxygen concentration reducing agent for anaerobic fermentation use according to this technique contains a saccharide oxidase as an active ingredient. Details of the saccharide oxidase are identical to those of the saccharide oxidase which can be employed in the method for producing a fermented food or beverage describe above and not described here.

The oxygen concentration reducing agent for anaerobic fermentation use may be constituted only from the saccharide oxidase described above as long as the saccharide oxidase described above is contained, or may be combined with one or more other ingredients which are selected as desired as long as the advantageous effects of this technique is not affected adversely. Other ingredients may be components employed ordinarily in formulation such as excipients, pH modifiers, colorants, corrigents, disintegrants, glidants, stabilizers and the like. It is also possible to use a component having a function which is known or will be discovered in future as desired depending on the purpose.

### <3. Anaerobic fermentation method>

The anaerobic fermentation method according to this technique is a method in which a saccharide oxidase action step and an anaerobic fermentation step are at least performed. Details of the saccharide oxidase action step and the anaerobic fermentation step performed in the anaerobic fermentation method according to this technique are identical to those of the saccharide oxidase action step and the anaerobic fermentation step in the method for producing a fermented food or beverage described above and not described here.

### Examples

The present invention is further detailed below on the basis of Examples. The following Examples are merely representatives of Examples of the invention, by which the scope of the present invention is not interpreted narrowly.

In this technique, as a measurement method for saccharide oxidase activity, the following measurement method was employed.

### [Glucose oxidase activity measurement method]

A suitable amount of an enzyme was taken and dissolved or dispersed uniformly in a pH 7.0 potassium phosphate-sodium hydroxide buffer solution (0.1 mol/L) to make 50 mL and used as a sample fluid. 2.50 g of D(+)-glucose was weighed and dissolved in water to make 25 mL, and used as a substrate solution. 0.5 mL of the substrate solution, 2 mL of the potassium phosphate-sodium hydroxide buffer solution (0.1 mol/L, pH 7.0, containing phenol), 0.5 mL of peroxidase test solution (25 units/mL) and 0.1 mL of 4-aminoantipyrine solution (1 in 250) were placed in a quartz cell and warmed at 37°C for 10 minutes. To this fluid, 0.1 mL of the sample fluid was added and mixed well and the warmed at 37°C and used as an assay fluid. Separately, the potassium phosphate-sodium hydroxide buffer solution (0.1 mol/L, pH 7.0) or water was employed instead of the sample fluid and processed similarly to the preparation of the assay fluid, thereby obtaining a comparison fluid. The assay fluid and the comparison fluid were measured for the absorbance at a wavelength of 500 nm 2 minutes and 5 minutes after addition of the sample fluid. Based on the molar extinction coefficient of a quinoneimine pigment which was generated, oxidized glucose was quantified. The amount of the enzyme required for oxidizing 1 µmol of glucose within 1 minute under the condition was regarded as one unit.

### <Experiment Example 1>

In Experimental Example 1, the behavior of the dissolved oxygen concentration in a milk raw material and the influence on the time for fermentation when using a saccharide oxidase for fermenting the milk raw material were investigated.

### (1) Experiment method

380 g of a commercially available cow milk ("Meiji Oishii Gyunyu"(solid-not-fat 8.3% or higher, milk fat 3.5% or higher), manufactured by Meiji Co., Ltd.), 11 g of a commercially available skimmed milk (manufactured by Morinaga Milk Industry Co., LTD.) and 57 g of water were mixed in a glass vessel to prepare a milk raw material mix, which was sterilized at 95°C for 5 minutes. Subsequently, each 30 mL of the sterilized milk raw material mix which was allowed to cool to room temperature was dispensed portionwise into a 50 mL tube in a clean bench, combined with an enzyme prepared by the method described in WO2014/042237 as an example of the saccharide oxidase in an amount of 5 to 120 U (based on the milk raw material mix) (0.17 to 4 U/mL), combined with 2v/v% of a lactic acid bacterium starter containing Lactobacillus delbrueckii subsp. bulgaricus and Streptococcus thermophilus, made miscible by inverting 5 times, and then fermented for several hours at 37°C in a thermostat water bath. The behaviors of the pH and the dissolved oxygen during the fermentation were monitored using a pH sensor and a DO sensor, respectively.

### (2) Results

The results are shown in Table 1.

**[Table 1]**

| Amount of saccharide oxidase added (U/mL) | Time after starting for reaching to dissolved oxygen level below 10% | Time for reaching to pH 5.5 |
|---|---|---|
| No addition | 2.5 hours | 2.8 hours |
| 0.17 | 0.3 hours | 2.3 hours |
| 0.4 | up to 0.2 hours | 2.2 hours |
| 0.8 | up to 0.2 hours | 1.9 hours |
| 4 | up to 0.2 hours | 1.8 hours |

### (3) Discussion

As shown in Table 1, the verification of the dissolved oxygen behavior indicated that the time for reaching to a dissolved oxygen level below 10% of that in the initiation of the fermentation in the no enzyme addition group was 2.5 hours while that in the saccharide oxidase addition group was shortened to 0.3 hours, showing that the enzyme treatment was capable of removing oxygen. In addition, the verification of the pH behavior indicated that the time for reaching to pH 5.5 at which the milk raw material forms a curd in the no enzyme addition group was 2.8 hours while that in the saccharide oxidase addition group was 2.3 hours at 0.17 U/mL and 1.9 hours at 0.8 U/mL or higher, showing that the time for fermentation was shortened by 1 hour at maximum. Based on these results, it became evident that the reduction in the oxygen concentration of the milk raw material by the action of the saccharide oxidase served to promote the anaerobic fermentation and to shorten the time for fermentation.

### <Experiment Example 2>

In Experiment Example 2, The advantageous effects of the simultaneous use of the saccharide oxidase and the lactase were verified.

### (1) Experiment method

As a lactase, Lactase Y "Amano" L-K (Kluyveromyces lactis-originated lactase) was employed. Similarly to Experiment Example 1, a sterilized milk raw material mix was prepared and dispensed into a 15 mL tube by 10 mL in a clean bench, and 0.8 U of saccharide oxidase (based on the milk raw material mix) (0.08 U/mL), 0.02 to 0.1v/v% of lactase agent (2 to 10 U/mL) and 2v/v% of lactic acid bacterium starter were added, made miscible by inverting 5 times, and then fermented for several hours at 37°C in a thermostat water bath. Each 0.5 mL was sampled every 1 hour to verify the pH of milk raw material mix.

### (2) Results

The results are shown in Table 2.

**[Table 2]**

| Amount of saccharide oxidase added (U/mL) | Amount of lactase added (U/mL) | pH after 4-hour fermentation | Physical state of fermented milk after 4 hours |
|---|---|---|---|
| No addition | No addition | 5.59 | Not solidified |
| 0.08 | No addition | 5.23 | Almost solidified, but slightly run down when inverting |
| 0.08 | 2 | 5.12 | Almost solidified, and never run down even when inverting |
| 0.08 | 5 | 5.2 | |
| 0.08 | 10 | 5.11 | |

### (3) Discussion

As shown in Table 2, the advantageous effects of simultaneous use of the enzyme were observed after 4-hour fermentation. While the treatment only with the saccharide oxidase allowed the curd to be formed after 4-hour fermentation but gave a somewhat loose physical state and a slight running down when inverting the tube, the treatment by simultaneous addition of the saccharide oxidase and the lactase gave no running down of the curd, showing a firmer physical state. Based on these results, the advantageous effects of simultaneous use of the saccharide oxidase and the lactase were observed.

### <Experiment Example 3>

In Experiment Example 3, the advantageous effects when performing treatment with the saccharide oxidase before fermentation step were verified.

### (1) Experiment method

Similarly to Experiment Example 1, a sterilized milk raw material mix was prepared, dispensed into a 15 mL tube by 10 mL in the clean bench, combined with no or 0.8 to 39.1 U of the saccharide oxidase (based on milk raw material mix) (0.08 to 3.91 U/mL), made miscible by inverting 5 times, and then allowed to react in a cool chamber at 5°C overnight (20 hours). The milk raw material mix combined with no enzyme or treated with 0.8 to 39.1 U of the saccharide oxidase (based on milk raw material mix) (0.08 to 3.91 U/mL) was sterilized at 100°C for 10 minutes, allowed to cool to room temperature, and then combined with 2v/v% of the lactic acid bacterium starter, made miscible by inverting 5 times, and then fermented for several hours at 37°C in a thermostat water bath. Each 0.5 mL was sampled every 1 hour to verify the pH of milk raw material mix.

### (2) Results

The results are shown in Table 3.

**[Table 3]**

| Amount of saccharide oxidase added (U/mL) | pH after 4-hour fermentation | Physical state of fermented milk after 4 hours |
|---|---|---|
| No addition | 5.58 | Not solidified |
| 0.08 | 5.43 | Solidified |
| 0.16 | 5.22 | |
| 0.39 | 5.34 | |
| 0.78 | 5.29 | |
| 3.91 | 5.18 | |

### (3) Discussion

As shown in Table 3, the time for fermentation was shortened in a manner dependent on the amount of the saccharide oxidase added even when the treatment with the saccharide oxidase was performed before the fermentation step, and it became evident that the advantageous effects can be exerted in both the cases before the fermentation and during the fermentation step.

### <Experiment Example 4>

In Experiment Example 4, the influence of the saccharide oxidase on the time for fermentation when employed during the fermentation of a plant-base raw material was investigated. As a plant-base raw material, a soybean milk was employed.

### (1) Experiment method

100 g of soybeans were combined with 400 g of tap water and immersed for 24 hours. Immersed soybeans were taken out, and 100 g was ground using a mixer (for about 2 minutes) thereby preparing a mashed soybean paste. To the mashed soybean paste thus obtained, water in an amount 7 times that of the mashed soybean paste was added while the mixer was used for finer grinding. The ground material was placed int a pot, brought into boiling, followed by pasteurizing with medium to low heat for 10 minutes while stirring with a spatula to avoid burning or sticking. After cooling, a filter bag was used to filter the mashed soybean paste, thereby preparing a soybean milk. The soybean milk thus prepared was dispensed into a 50 mL tube by 30 mL, and sterilized for 5 minutes at 95°C.

The sterilized soybean milk was cooled with water, and 0.6 mL of the lactic acid bacterium starter containing Lactobacillus delbrueckii subsp. delbrueckii and 0.24 U (0.008 U/mL) of the saccharide oxidase employed in Experiment Example 1 described above were added in the clean bench and fermented overnight at 37°C. During fermentation, the pH was monitored using a pH sensor. The case where no saccharide was added was referred to as no enzyme addition group.

### (2) Results

The results are shown in Table 4.

**[Table 4]**

| Amount of saccharide oxidase added (U/mL) | Time for reaching to pH 4.5 | Shortened time |
|---|---|---|
| No addition | 9.3 hours | - |
| 0.008 U/mL | 8.2 hours | 1.1 hours |

### (3) Discussion

As shown in Table 4, the no enzyme addition group required 9.3 hours for reaching to pH 4.5 at which the curd of the soybean milk raw material was formed, while the saccharide oxidase addition group required 8.2 hours, exhibiting a shortening of the time for fermentation by 1.1 hours. Based on these results, it became evident that the time for fermentation can be shortened by using this technique also when using the soybean milk which is a plant-base raw material.

### <Experiment Example 5>

In Experiment Example 5, the influence of the saccharide oxidase on the time for fermentation when employed during the fermentation using an oat milk as a plant-base raw material was investigated.

### (1) Experiment method

An oat milk ("oat milk" manufactured by Oatly Group AB) was dispensed in 10 mL-portions, and 0.2 mL of the lactic acid bacterium starter containing Lactobacillus delbrueckii subsp. delbrueckii and 0.08 U (0.008 U/mL) of the saccharide oxidase employed in Experiment Example 1 described above were added in the clean bench and fermented overnight at 37°C. During fermentation, the pH was monitored using a pH sensor. The case where no saccharide was added was referred to as no enzyme addition group.

### (2) Results

The results are shown in Table 5.

**[Table 5]**

| Amount of saccharide oxidase added (U/mL) | Time for reaching to pH 4.5 | Shortened time |
|---|---|---|
| No addition | 7.8 hours | - |
| 0.008 U/mL | 5.5 hours | 2.3 hours |

### (3) Discussion

As shown in Table 5, the no enzyme addition group required 7.8 hours for reaching to pH 4.5 at which the curd of the oat milk raw material was formed, while the saccharide oxidase addition group required 5.5 hours, exhibiting a shortening of the time for fermentation by 2.3 hours. Based on these results, it became evident that the time for fermentation can be shortened by using this technique also when using the oat milk which is a plant-base raw material.

### <Experiment Example 6>

In Experiment Example 6, the influence of the saccharide oxidase on the spinnability when employed during the fermentation of a plant-base raw material was investigated. As a plant-base raw material, a soybean milk was employed.

### (1) Experiment method

To a sterilized 300-mL beaker, 280 mL of a soybean milk (Manufactured by MARUSAN-AI CO., LTD. "Organic ingredient-unadjusted soy milk") was dispensed and cooled with water, and then 5.6 mL of the lactic acid bacterium starter containing Lactobacillus delbrueckii subsp. delbrueckii and 1.5 U/mL of the saccharide oxidase employed in Experiment Example 1 described above or 2.0 U/mL of a cellulase were added in the clean bench and fermented overnight at 37°C. After fermentation, the spinnability of the soybean milk yogurt was evaluated by measuring the flowing time period using a Zahn Cup. Specifically, the time period until the termination of the flow of a sample flowing from the orifice of the Zahn Cup was regarded as the flowing time period. The case where no saccharide was added was referred to as no enzyme addition group.

### (2) Results

The results are shown in Table 6.

**[Table 6]**

| Enzyme | Flowing time period |
|---|---|
| No addition | 6 seconds |
| 1.5 U/mL Saccharide oxidase | 100 seconds |
| 2 U/mL Cellulase | 30 seconds |

### (3) Discussion

As shown in Table 6, the no enzyme addition group exhibited a flowing time period of 6 seconds, while the saccharide oxidase addition group exhibited a flowing time period of 100 seconds, showing a marked improvement in the spinnability. Although the cellulase addition group exhibited an improvement in the spinnability when compared with the no enzyme addition group, the spinnability-improving effect was more significant in the saccharide oxidase addition group. Based on these results, it became evident that the spinnability can be improved by using this technique also even when using the plant-base raw material.

### <Experiment Example 7>

In Experiment Example 7, the influence of the amount of the saccharide oxidase added on the spinnability of a plant-base raw material after fermentation was investigated. As a plant-base raw material, a soybean milk was employed.

### (1) Experiment method

To a sterilized 300-mL beaker, 280 mL of a soybean milk (Manufactured by MARUSAN-AI CO., LTD. "Organic ingredient-unadjusted soy milk") was dispensed and cooled with water, and then 5.6 mL of the lactic acid bacterium starter containing Lactobacillus delbrueckii subsp. delbrueckii and the saccharide oxidase employed in Experiment Example 1 in an amount indicated in Table 7 shown below were added in the clean bench and fermented overnight at 37°C. After fermentation, the spinnability of the soybean milk yogurt was evaluated by measuring the flowing time period using a Zahn Cup.

### (2) Results

The results are shown in Table 7.

**[Table 7]**

| Amount of saccharide oxidase added (U/mL) | Flowing time period |
|---|---|
| 0.015 | 13 seconds |
| 0.15 | 13 seconds |
| 1.5 | 100 seconds |
| 15 | 165 seconds |

### (3) Discussion

As shown in Table 7, the flowing time period was prolonged markedly when the saccharide oxidase was added at 1.5 U/mL or higher when compared with the cases of 0.015 U/mL and 0.15 U/mL. Based on these results, it is preferable to add the saccharide oxidase at 1.5 U/mL or higher in order to ensure the improvement in the spinnability.

## Claims

1. A method for producing a fermented food or beverage, comprising a saccharide oxidase action step for allowing a saccharide oxidase to act on a portion or the whole of a saccharide in a raw material; and, an anaerobic fermentation step for performing anaerobic fermentation.

2. The method for producing a fermented food or beverage according to claim 1, wherein the saccharide oxidase action step is performed before the anaerobic fermentation step and/or simultaneously with the anaerobic fermentation step.

3. The method for producing a fermented food or beverage according to claim 1 or 2, wherein the saccharide oxidase has a property of acting on one or more saccharides selected from glucose, maltotriose, maltose, galactose, maltotetraose, lactose, cellobiose, and maltodextrin.

4. The method for producing a fermented food or beverage according to any one of claims 1 to 3, wherein the saccharide oxidase consists of a polypeptide represented by any of the following (1) to (3):
(1) a polypeptide consisting of the amino acid sequence represented by SEQ ID NO:1,
(2) a polypeptide which is formed as a result of substitution, addition, insertion or deletion of one or several amino acid residues in the amino acid sequence represented by SEQ ID NO:1 and which exhibits a substrate specificity equivalent to that of the polypeptide consisting of the amino acid sequence represented by SEQ ID NO:1; and,
(3) a polypeptide which has a sequence identity of 90% or more to the amino acid sequence represented by SEQ ID NO: 1 in the amino acid sequence represented by SEQ ID NO: 1 and which exhibits a substrate specificity equivalent to that of the polypeptide consisting of the amino acid sequence represented by SEQ ID NO:1.

5. The method for producing a fermented food or beverage according to any one of claims 1 to 4, wherein in the anaerobic fermentation step, a lactic acid fermentation is performed.

6. The method for producing a fermented food or beverage according to any one of claims 1 to 5, wherein the fermented food or beverage is a fermented milk.

7. An oxygen concentration reducing agent for anaerobic fermentation use, which comprises a saccharide oxidase.

8. An anaerobic fermentation method, comprising a saccharide oxidase action step for allowing a saccharide oxidase to act on a portion or the whole of a saccharide in a raw material.

9. The anaerobic fermentation method according to claim 8, wherein the saccharide oxidase action step is performed before an anaerobic fermentation step and/or simultaneously with the anaerobic fermentation step.
